# EUROPÄISCHE PATENTANMELDUNG

(11) **EP 3 330 058 A1**
(43) Veröffentlichungstag der Anmeldung: **06.06.2018**
(21) Anmeldenummer: 17208680.3
(22) Anmeldetag: 24.04.2014
(51) Int. Cl.: B29C 49/42, B29L 31/00, B29C 49/06, A61L 2/18, A61L 2/20, A61L 2/22, B67C 7/00, A61L 2/00, A61L 9/00, B29L 31/16

(54) **VORRICHTUNG UND VERFAHREN ZUR AUSSENSTERILISATION VON KUNSTSTOFFVORFORMLINGEN**

(30) Priorität: 24.04.2013 DE 102013104152
(62) Teilanmeldung aus: 14001461.4
(71) Anmelder: Krones AG, 93073 Neutraubling (DE)
(72) Erfinder: Seidenberg, Katharina, 93073 Neutraubling (DE); Kohl, Bettina, 93073 Neutraubling (DE); Loy, Michael, 93073 Neutraubling (DE); Söllner, Jürgen, 93073 Neutraubling (DE); Goldbach, Marina, 93073 Neutraubling (DE); Schmitt, Robert, 93073 Neutraubling (DE); Bedoe, Ute, 93073 Neutraubling (DE)
(74) Vertreter: Bittner, Bernhard

(57) **Zusammenfassung**

Vorrichtung (1) zum Sterilisieren von Kunststoffbehältnissen (10) und insbesondere von Kunststoffvorformlingen (10), mit einer Transporteinrichtung (2), welche die Kunststoffbehältnisse (10) entlang eines vorgegebenen Transportpfades transportiert, mit wenigstens einer Beaufschlagungseinrichtung (4), welche eine Außenoberfläche der zu sterilisierenden Kunststoffbehältnisse mit einem fließfähigen Medium beaufschlagt. Erfindungsgemäß ist die Beaufschlagungseinrichtung derart angeordnet, dass sie einen vorgegebenen, unterhalb einer Mündung der Kunststoffvorformlinge liegenden Bereich der Kunststoffbehältnisse (10) mit dem Sterilisationsmedium beaufschlagt.

## Beschreibung

Die vorliegende Erfindung bezieht sich auf eine Vorrichtung und ein Verfahren zur Außensterilisation von Kunststoffbehältnissen und insbesondere von Kunststoffvorformlingen. Um die Haltbarkeit von insbesondere sensiblen Füllprodukten in Kunststoffbehältnissen, wie beispielsweise PET-Flaschen, entscheidend zu verbessern, ist es bekannt, vor dem Füllprozess die Anzahl der Keime in den Behältnissen deutlich zu reduzieren. Dazu sind im Stand der Technik und im Bereich der Fülltechnik verschiedene Nass- und Trockenaseptikmethoden bekannt. Dabei ist es beispielsweise bekannt, dass in das Innere der Kunststoffbehältnisse ein Sterilisationsmedium wie Wasserstoffperoxid oder Peressigsäure eingeführt wird. Auch sind Verfahren bekannt, bei denen Elektronenstrahlenemitter in den Innenraum der Kunststoffbehältnisse eingeführt werden. Die Erfindung wird hier unter Bezugnahme auf Kunststoffbehältnisse beschrieben, es wird jedoch darauf hingewiesen, dass auch eine Anwendung auf andere Behältnisse wie beispielsweise Glasbehältnisse in Betracht kommt.

Aufgrund der teilweise großen Volumina derartiger Behältnisse entsteht jedoch bei dem Einsatz von Sterilisationsmedien ein hoher Verbrauch derselben.

Daher sind aus dem Stand der Technik Vorrichtungen und Verfahren bekannt, welche nicht die bereits fertig ausgeformten Behältnisse sterilisieren, sondern bereits die Kunststoffvorformlinge, welche anschließend zu den Kunststoffbehältnissen umgeformt werden. Mit anderen Worten wird die Anzahl der Keime bereits vor dem Blasen der Behältnisse innerhalb des Kunststoffvorformlings (im Folgenden auch als Preform bezeichnet) reduziert. Dabei durchläuft dieser Kunststoffvorformling einen Behandlungsbereich, in dem die Entkeimung durch gasförmige oder flüssige Sterilisationsmedien oder durch Bestrahlung (UV, Elektronenstrahl und dergleichen) erzielt wird.

Nach diesem Entkeimungsvorgang sollte vorteilhaft bis zum Verschließen des befüllten Behälters eine Wiederverkeimung verhindert werden. Idealerweise wird dazu der Umformungsvorgang der Kunststoffvorformlinge unter sterilen Bedingungen durchgeführt. Um dort die sterilen Bedingungen während der Produktion aufrecht zu erhalten, dürfen durch die Kunststoffvorformlinge keine Keime in den sterilen Bereich transportiert werden. Diese könnten sich dort absetzen und danach in einen nachfolgenden Vorformling gelangen.

Der vorliegenden Erfindung liegt daher die Aufgabe zugrunde, - insbesondere neben den Innenflächen - auch die Außenflächen der Kunststoffvorformlinge zu entkeimen. Diese Aufgabe wird erfindungsgemäß durch die Gegenstände der unabhängigen Ansprüche erreicht. Vorteilhafte Ausführungsformen und Weiterbildungen sind Gegenstand der Unteransprüche.

Eine erfindungsgemäße Vorrichtung zum Sterilisieren von Kunststoffbehältnissen und insbesondere Kunststoffvorformlingen weist eine Transporteinrichtung auf, welche die Kunststoffbehältnisse entlang eines vorgegebenen Transportpfades transportiert. Weiterhin weist die Vorrichtung wenigstens eine Beaufschlagungseinrichtung auf, welche eine Außenoberfläche der zu sterilisierenden Kunststoffbehältnisse mit einem fließfähigen (insbesondere einem gasförmigen und/oder flüssigen) Medium beaufschlagt.

Erfindungsgemäß ist die Beaufschlagungseinrichtung derart angeordnet, dass sie einen vorgegebenen unterhalb einer Mündung des Kunststoffvorformlings liegenden Bereich der Kunststoffbehältnisse mit dem Sterilisationsmedium beaufschlagt.

Die Anmelderin hat herausgefunden, dass im Rahmen derartiger Sterilisationsvorgänge ein besonders kritischer Bereich gerade die Außenfläche der Kunststoffvorformlinge ist, da die dort vorhandenen Keime sich in unmittelbarer Nähe zur Innenoberfläche befinden. Dadurch besteht ein relativ hohes Risiko, dass diese Keime während der auf die Entkeimung folgenden Prozessschritte in den Vorformling bzw. Behälter und/oder in nachfolgende Vorformlinge gelangen können. Daher kommt der Entkeimung der Außenflächen des Kunststoffvorformlings entgegen früheren Annahmen eine besondere Bedeutung zu. Bei einer aus dem internen Stand der Technik der Anmelderin bekannt gewordenen Anlage wird eine Entkeimungsleistung dadurch erzielt, dass die Kunststoffvorformlinge während der Innenentkeimung durch einen Behandlungsbereich transportiert werden, in dessen Umgebung eine erhöhte Konzentration des Sterilisationsmediums herrscht. Die so erzielten Entkeimungsraten an den Außenflächen haben sich jedoch gerade bei sensiblen Füllprodukten, wie beispielsweise schwachsauren Getränken, als nicht ausreichend erwiesen.

In den unten beschriebenen bevorzugten Ausführungsformen sollen daher auch Lösungsmöglichkeiten für eine verbesserte Entkeimung der Außenflächen beschrieben werden.

Bei einer bevorzugten Ausführungsform handelt es sich bei dem Sterilisationsmedium um ein gasförmiges Medium, beispielsweise um Wasserstoffperoxid oder Peressigsäure. Bevorzugt transportiert die Transporteinrichtung die Kunststoffbehältnisse, bei denen es sich insbesondere um Kunststoffvorformlinge handelt, in einer horizontalen Ebene. Bevorzugt transportiert die Transporteinrichtung die Kunststoffvorformlinge entlang eines kreisförmigen Transportpfades. Bei einer weiteren vorteilhaften Ausführungsform weist die Vorrichtung eine weitere Sterilisationseinrichtung auf, welche auch die Innenoberflächen der Kunststoffvorformlinge sterilisiert. Dabei kann es sich sowohl um eine Vorrichtung handeln, welche diese Innenoberflächen der Kunststoffvorformlinge mit dem Sterilisationsmedium beaufschlagt, es wäre jedoch auch denkbar, dass diese weitere Sterilisationseinrichtung die Innenoberflächen der Kunststoffvorformlinge mit Strahlung, insbesondere UV- oder Elektronenstrahlung, beaufschlagt.

Bei einer weiteren vorteilhaften Ausführungsform weist die Vorrichtung eine Umformungseinrichtung auf, welche Kunststoffvorformlinge zu Kunststoffbehältnissen umformt. Vorteilhaft weist diese Umformungseinrichtung einen Reinraum bzw. Sterilraum auf, innerhalb dessen die Kunststoffvorformlinge zu den Kunststoffbehältnissen umgeformt werden. Dabei weist diese Umformungseinrichtung bevorzugt auch eine Beaufschlagungseinrichtung auf, welche die Kunststoffvorformlinge zum Zwecke ihrer Expansion mit einem gasförmigen Medium beaufschlagt und insbesondere mit Sterilluft beaufschlagt.

Bei einer weiteren vorteilhaften Ausführungsform weist die Vorrichtung auch eine Erwärmungseinrichtung auf, welche die Kunststoffvorformlinge erwärmt. Die hier beschriebenen Sterilisationseinrichtungen sind bevorzugt in der Transportrichtung der Kunststoffvorformlinge stromabwärts bezüglich der Erwärmungseinrichtung angeordnet, also insbesondere zwischen der Erwärmungseinrichtung und der Umformungseinrichtung. Es wäre jedoch auch denkbar, dass die Außen- und/oder Innensterilisation der Kunststoffvorformlinge bereits vor der Erwärmung stattfindet und auch die Erwärmungseinrichtung einen Reinraum bzw. Sterilraum aufweist, durch den die Kunststoffvorformlinge während ihrer Erwärmung transportiert werden. Auch die (Vor-)Sterilisation der Kunststoffvorformlinge innerhalb der Erwärmungseinrichtung wäre denkbar.

Bei einer weiteren vorteilhaften Ausführungsform ist die oder wenigstens eine Beaufschlagungseinrichtung unterhalb des Transportpfades der Kunststoffvorformlinge angeordnet. Hierunter ist zu verstehen, dass die Beaufschlagungseinrichtung bevorzugt unterhalb eines Bodenabschnitts der Kunststoffvorformlinge angeordnet ist oder unterhalb einer Ebene, oberhalb derer die Kunststoffvorformlinge gefördert werden. Es wird darauf hingewiesen, dass aus dem Stand der Technik auch Vorrichtungen bekannt sind, bei denen die Kunststoffvorformlinge mit ihrer Mündung nach unten transportiert werden. In diesem Falle ist die Beaufschlagungseinrichtung entsprechend derart angeordnet, dass sie einen vorgegebenen, oberhalb der Mündung der Kunststoffvorformlinge liegenden Bereich der Kunststoffvorformlinge mit dem Sterilisationsmedium beaufschlagt.

Unter dem Begriff "unterhalb" wird daher die Situation verstanden, bei der der Kunststoffvorformling aufrecht stehend mit der Mündung nach oben angeordnet ist. Daneben kann unter der Transportebene der Kunststoffvorformlinge auch diejenige Ebene verstanden werden, die durch die Mündung der Kunststoffvorformlinge bzw. den oberen Mündungsrand der Kunststoffvorformlinge definiert ist. Damit wird bei dieser Ausgestaltung vorgeschlagen, dass wenigstens eine Austrittsöffnung der Beaufschlagungseinrichtung, durch welche das Sterilisationsmedium auf die Kunststoffvorformlinge trifft, unterhalb des Transportpfades bzw. zumindest unterhalb eines Höhenniveaus eines Mündungsabschnitts der Kunststoffvorformlinge angeordnet ist. Es wird darauf hingewiesen, dass es hierbei auch denkbar wäre, dass die Beaufschlagungseinrichtungen teilweise oder auch vollständig oberhalb dieses Niveaus angeordnet sind, etwa, wenn Umlenkeinrichtungen vorgesehen sind, welche das Sterilisationsmedium (ausschließlich) auf einen Bereich unterhalb der Mündung und/oder eines Tragrings der Kunststoffvorformlinge richten.

Vorteilhaft sind die Beaufschlagungseinrichtungen unterhalb der transportierten Kunststoffvorformlinge angeordnet. Auf diese Weise wird insbesondere auch ein Bodenbereich der Kunststoffvorformlinge mit dem Sterilisationsmedium beaufschlagt. Es wäre jedoch auch denkbar, dass die Beaufschlagungseinrichtungen seitlich neben dem Transportpfad der Kunststoffbehältnisse angeordnet sind.

Vorteilhaft ist wenigstens eine Beaufschlagungseinrichtung derart angeordnet, dass aus ihr austretendes Sterilisationsmedium wenigstens teilweise in eine Richtung strömt, die auch eine Komponente aufweist, die sich in der Längsrichtung der Kunststoffvorformlinge von deren Bodenbereich zu deren Mündungsbereich erstreckt.

Bei einer weiteren vorteilhaften Ausführungsform ist wenigstens eine Beaufschlagungseinrichtung der Vorrichtung (gegenüber den Behältnisse) höhenverstellbar d.h. die Relativposition wenigstens einer Beaufschlagungseinrichtung kann in einer Längsrichtung der Behältnisse relativ zu diesen verändert werden. Dabei wäre es möglich und bevorzugt, dass eine Position der Beaufschlagungseinrichtungen verändert wird, es wäre jedoch auch eine Veränderung der Position der Behältnisse denkbar. Auf diese Weise kann eine Anpassung an unterschiedliche Höhen bzw. Geometrien der zu sterilisierenden Kunststoffvorformlinge ermöglicht werden.

Bei einer weiteren vorteilhaften Ausführungsform weist die Vorrichtung eine Vielzahl von Beaufschlagungseinrichtungen auf. Diese können dabei an einen gemeinsamen Träger angeordnet sein. Dabei ist es beispielsweise möglich, dass ein Ringkanal den einzelnen Beaufschlagungseinrichtungen das Sterilisationsmedium zuführt und diese damit die Kunststoffvorformlinge beaufschlagen.

Bei einer weiteren vorteilhaften Ausführungsform weist die Vorrichtung eine erste Wandung auf, welche sich in einer Richtung des Transportpfades erstreckt und welche bevorzugt seitlich des Transportpfades angeordnet ist. Bei einer Lösungsmöglichkeit zur Erhöhung der Konzentration des gasförmigen Sterilisationsmediums - insbesondere in unmittelbarer Umgebung der Außenoberfläche des Kunststoffvorformlings - wird daher vorgeschlagen, den Behandlungsbereich für die Kunststoffvorformlinge bzw. den Behandlungsbereich um den Transportpfad der Kunststoffvorformlinge zu verkleinern. Dazu ist es beispielsweise möglich, diesen Transportpfad durch die hier beschriebene Wand einzugrenzen. Allgemein wäre es vorteilhaft, den Transportpfad ganz oder teilweise durch Wandungen, wie beispielsweise gebogene Leitbleche, einzuhausen. Dabei ist es möglich, dass diese Wandungen bezüglich des Transportpfades außen oder innen oder beispielsweise auch U-förmig um den Transportpfad angebracht werden.

Bevorzugt weist die Vorrichtung daher eine zweite Wandung auf, wobei der Transportpfad der Kunststoffvorformlinge zwischen der ersten Wandung und der zweiten Wandung angeordnet ist. Bevorzugt weist die Vorrichtung daher einen Kanal auf, innerhalb dessen die Kunststoffvorformlinge transportiert werden und innerhalb dessen sie bevorzugt auch mit dem fließfähigen Sterilisationsmedium beaufschlagt werden.

Bei einer weiteren vorteilhaften Ausführungsform weist die Vorrichtung eine Vielzahl von Beaufschlagungseinrichtungen auf, die entlang des Transportpfades der Kunststoffbehältnisse hintereinander angeordnet sind. Auf diese Weise ist es auch möglich, eine Vielzahl von Kunststoffvorformlingen im Wesentlichen gleichzeitig mit dem Sterilisationsmedium an ihrer Außenoberfläche zu beaufschlagen. Dabei ist es möglich, dass diese Beaufschlagungseinrichtungen stationär angeordnet sind. Es wäre jedoch auch denkbar, dass sich diese Beaufschlagungseinrichtungen mit den Kunststoffvorformlingen mitbewegen oder sich beispielsweise auch entgegen der Transportrichtung der Kunststoffvorformlinge bewegen.

Bei einer weiteren vorteilhaften Ausführungsform weist die Vorrichtung eine Strömungserzeugungseinrichtung auf, welche in dem Bereich des Transportpfades eine Strömung des fließfähigen Mediums erzeugt. Dabei kann es sich beispielsweise um eine aktive Einrichtung handeln, wie etwa eine Ventilationseinrichtung, welche eine Strömung an der Außenoberfläche der Kunststoffvorformlinge erzeugt. Insbesondere soll auf diese Weise in der Umgebung der Kunststoffvorformlinge eine (insbesondere turbulente) Strömung des Sterilisationsmediums erzeugt werden.

Bei dieser Vorgehensweise wird daher vorgeschlagen, den Entkeimungsvorgang dadurch zu verbessern, dass der Stofftransport durch eine gezielte Umströmung der zu entkeimenden Außenoberflächen verbessert wird. Diese Strömung kann beispielsweise durch einen Ventilator, der sich in dem Behandlungsbereich befindet und in Richtung des Transportpfades der Kunststoffvorformlinge ausgerichtet ist, erreicht werden. Daneben könnte auch ein entsprechendes Gebläse zum Einsatz kommen. Der Ventilator bzw. das Gebläse kann dabei einen separaten Antrieb aufweisen, der sich bevorzugt außerhalb des Behandlungsbereiches befindet. Alternativ oder zusätzlich wäre es auch denkbar, die Antriebsleistung über ein entsprechendes Getriebe von der Drehbewegung der Transportsterne bzw. der Transporteinrichtung abzuzweigen.

Daneben wäre es auch denkbar, die Umströmung der Kunststoffvorformlinge mithilfe einer oder mehrerer Düsen zu erzeugen. Dabei ist es denkbar, dass die Düse oder die Düsen vorzugsweise mit dem gasförmigen Sterilisationsmedium durchströmt werden. Es ist dabei insbesondere denkbar, dass die Düsen in einer Ausführungsform fest in dem Behandlungsbereich installiert sind und in Richtung des Transportpfades der Kunststoffvorformlinge ausgerichtet sind. Bei einer besonderen Ausführungsform sind dabei mehrere Beaufschlagungseinrichtungen bzw. Düsen zumindest teilweise entlang des Transportpfades unterhalb oder seitlich der Kunststoffvorformlinge angeordnet. Dabei ist es, insbesondere bei der Ausführung der Anordnung unterhalb der Kunststoffvorformlinge, auch denkbar, dass das Höhenniveau der Austrittsöffnungen der Beaufschlagungseinrichtungen an die Länge der Kunststoffvorformlinge angepasst werden kann, beispielsweise durch die oben beschriebene Höhenverstellung, aber gegebenenfalls auch durch unterschiedlich lange Aufsätze für die Beaufschlagungseinrichtungen.

Alternativ können die Beaufschlagungseinrichtungen auch mit dem Kunststoffvorformling zumindest teilweise entlang des Transportpfades in dem Behandlungsbereich mitbewegt werden. Dabei ist es denkbar, dass die Beaufschlagungseinrichtungen wiederum unterhalb oder seitlich an dem Kunststoffvorformling bzw. dessen Transportpfad angeordnet sind. Auch bei derartigen mitbewegten Beaufschlagungseinrichtungen ist eine Anpassung des Höhenniveaus an die Lage, bzw. Länge der Kunststoffvorformlinge durch einen Verstellmechanismus oder verschiedene Aufsatzdüsen möglich. Daneben wäre es auch denkbar, einzelne Lösungsmöglichkeiten kombiniert einzusetzen, beispielsweise die Düsen kombiniert mit einer Einhausung.

Bei einer weiteren vorteilhaften Ausführungsform ist damit eine Vielzahl von Beaufschlagungseinrichtungen an einem gemeinsamen Ring, beispielsweise einem Düsenring, angeordnet. Dabei kann beispielsweise an der Außenoberfläche der Kunststoffvorformlinge außerhalb der laminaren Grenzschicht eine turbulente Strömung erzeugt werden, um den Stoffaustausch zu erhöhen. Auf diese Weise können in kürzerer Zeit mehr Keime abgetötet werden.

Bei einer bevorzugten Ausführungsform sind daher Möglichkeiten vorhanden, welche in der Umgebung der Kunststoffvorformlinge eine turbulente Strömung des Sterilisationsmediums sicherstellen. Mit anderen Worten ist bevorzugt das Sterilisationsmedium im Bereich der Kunststoffvorformlinge turbulent. Auf diese Weise kann in kürzerer Zeit eine verbesserte Entkeimung ermöglicht werden. Bei einer Variante sind die Beaufschlagungseinrichtungen an einem gemeinsamen Träger angeordnet und dieser Träger kann entgegen der Transportrichtung der Kunststoffvorformlinge bewegt werden. Vorteilhaft handelt es sich hierbei um einen drehbaren Träger, sodass ein derartiger Träger rotiert werden kann. Auch die Transporteinrichtung zum Transportieren der Kunststoffvorformlinge ist bei dieser bevorzugten Ausführungsform bevorzugt ein drehbares Rad oder weist ein solches auf. Bevorzugt sind daher allgemein Mittel vorgesehen, welche eine Turbulenz des Sterilisationsmediums im Bereich der Kunststoffvorformlinge fördern und zwar insbesondere Mittel, welche eine Turbulenz erzeugen bzw. diejenige Turbulenz erhöhen, welche sich aus der bloßen Relativbewegung der Kunststoffvorformlinge gegenüber ihrer Umgebung ergibt. Dabei wäre es beispielsweise möglich, dass Einrichtungen vorgesehen sind, welche eine Strömungsänderung des Sterilisationsmediums insbesondere auch im Bereich der Kunststoffvorformlinge ermöglichen.

Dies hätte den Vorteil, dass ein Sterilisationsmedium, welches bevorzugt turbulent aus der Beaufschlagungseinrichtung auftritt, aktiv verwirbelt wird, da der Transport der Kunststoffvorformlinge eine Strömung in eine erste Richtung und der Träger der Beaufschlagungseinrichtung eine Strömung in eine diesbezüglich, das heißt bezüglich der ersten Richtung entgegengesetzte Richtung, erzeugt. Zusätzlich kann eine Drehgeschwindigkeit des Trägerrings für die Beaufschlagungseinrichtungen derart angepasst bzw. eingestellt werden, dass unter anderem abhängig der Geometrie der Kunststoffvorformlinge immer eine optimale turbulente Strömung im Umfeld der Außenoberfläche der Kunststoffvorformlinge erzeugt wird, bzw. das Vorhandensein einer turbulenten Strömung unterstützt wird. Alternativ oder zusätzlich ist es auch denkbar, dass der Düsenring in seiner Höhe automatisch anhand der Geometrie der Kunststoffvorformlinge verstellt wird, um die Strömungsverhältnisse zu beeinflussen.

Bei einer weiteren vorteilhaften Ausführungsform weist die Vorrichtung eine Umlenkeinrichtung auf, welche eine Strömung des fließfähigen Sterilisationsmediums umlenkt. Dabei ist es beispielsweise möglich, dass ein vorgegebener Anteil des aus der Beaufschlagungseinrichtung austretenden Sterilisationsmediums (auch) auf diese Umlenkeinrichtung gelangt und auf diese Weise auch bevorzugt auf eine Außenoberfläche der Kunststoffvorformlinge abgelenkt wird. Dabei ist es denkbar, dass eine derartige Strömungsumlenkeinrichtung fest angeordnet ist und den Gasstrom derart umleitet, dass ein Kunststoffvorformling umfassend mit dem Sterilisationsmedium benetzt wird. Dabei ist es denkbar, dass eine derartige Umlenkeinrichtung innerhalb eines Transportpfades der Kunststoffvorformlinge (insbesondere bei einem kreisförmigen Transportpfad) angebracht ist.

Daneben ist es auch möglich, dass eine derartige Umlenkeinrichtung in einem vorgegebenen Winkel (z.B. bezüglich einer Längsrichtung der Kunststoffvorformlinge) angeordnet ist und den darauf auftreffenden Strahl des gasförmigen Sterilisationsmediums umlenkt, um eine breite Benetzung des Kunststoffvorformlings zu erreichen. Auch wäre es denkbar, dass eine derartige Umlenkeinrichtung gewinkelt ausgeführt ist, beispielsweise derart, dass der Gasstrom des Sterilisationsmediums mehrfach auf den Kunststoffvorformling auftrifft.

Diese Umlenkeinrichtung kann dabei stationär angeordnet sein, es wäre jedoch auch denkbar, dass sie sich mit den Beaufschlagungseinrichtungen und/oder den zu sterilisierenden Kunststoffvorformlingen mitbewegt.

Bei einer weiteren vorteilhaften Ausführungsform ist, wie oben beschrieben, eine weitere Vorrichtung zum Sterilisieren von Innenoberflächen des Kunststoffvorformlings angeordnet, wobei diese bevorzugt so vorgesehen ist, dass zumindest zeitweise eine gleichzeitige Innen- und Außensterilisation der Kunststoffvorformlinge möglich ist.

In diesem Zusammenhang wird darauf hingewiesen, dass physikalisch betrachtet auf der Außenoberfläche der Kunststoffvorformlinge selbst keine turbulente Strömung entsteht. In einer laminaren Grenzschicht um die Kunststoffvorformlinge herrscht immer eine laminare Strömung, weil aufgrund der Fluidreibung die Geschwindigkeit direkt an der Oberfläche des Kunststoffvorformlings Null ist und dann mit zunehmendem Abstand zur Oberfläche zunimmt. Bei einem bestimmten Abstand wird die sogenannte kritische Reynoldszahl überschritten und die Strömung ist dann turbulent. In unmittelbarer Umgebung der Außenoberfläche tritt damit ein Stofftransport durch Diffusion und außerhalb der laminaren Grenzschicht eine schneller ablaufende Diffusion durch die Wirbel in der Strömung auf.

Bei einer weiteren vorteilhaften Ausführungsform sind Beaufschlagungseinrichtungen zum Beaufschlagen der Innenoberflächen der Kunststoffvorformlinge vorgesehen, die bevorzugt mit den Kunststoffvorformlingen beweglich ausgebildet sind, wobei die Beaufschlagungseinrichtungen derart angeordnet sind, dass sie das fließfähige Medium unter einem vorgegebenen von 0° verschiedenen Winkel gegenüber einer Längsrichtung der Kunststoffvorformlinge in diese einspritzen und/oder die Längsrichtungen der Beaufschlagungseinrichtungen gegenüber den Längsrichtungen der Kunststoffvorformlinge versetzt angeordnet sind. Auf diese Weise wird auch eine höchst effiziente Innensterilisation der Kunststoffvorformlinge erreicht, da auch im Inneren der Kunststoffvorformlinge Verwirbelungen des Sterilisationsmediums bzw. dessen Strömung erzeugt werden.

Dabei ist es auch möglich, dass eine Einspritzeinrichtung des Sterilisationsmediums eine Komponente aufweist, welche entgegen der Transportrichtung der Kunststoffvorformlinge ausgerichtet ist.

Bei einer weiteren vorteilhaften Ausführungsform weist die Vorrichtung eine weitere Sterilisationseinrichtung auf, welche eine Innenoberfläche der Kunststoffvorformlinge sterilisiert. Dabei kann es sich um eine Sterilisationseinrichtung handeln, welche die Kunststoffvorformlinge zu deren Sterilisation ebenfalls mit einem fließfähigen Medium beaufschlagt, es kann jedoch auch eine Sterilisationseinrichtung zur Anwendung kommen, welche diese Innenoberflächen mit Strahlung, beispielsweise mit einer Ladungsträgerstrahlung und insbesondere mit einer Elektronenstrahlung beaufschlagt.

Die vorliegende Erfindung ist weiterhin auf ein Verfahren zum Sterilisieren von Kunststoffvorformlingen gerichtet, wobei die Kunststoffvorformlinge entlang eines vorgegebenen Transportpfades transportiert werden und während dieses Transports wenigstens ein Bereich einer Außenoberfläche der Kunststoffvorformlinge mit einem fließfähigen (d.h. insbesondere gasförmigen und/oder flüssigen) Sterilisationsmedium beaufschlagt wird. Erfindungsgemäß wird von wenigstens einer Beaufschlagungseinrichtung wenigstens ein Bereich der Außenoberfläche der Kunststoffvorformlinge beaufschlagt, der unterhalb eines Mündungsbereiches der Kunststoffvorformlinge liegt. Dabei wird unter "unterhalb" wiederum verstanden, dass dieser Bereich sich zwischen einem Mündungsbereich und einem Bodenbereich des Kunststoffvorformlings befindet, wobei "unterhalb" insbesondere im Hinblick auf eine aufrechtstehende Position des Kunststoffvorformlings (mit der Mündung nach oben) zu verstehen ist. Vorteilhaft wird daher auch ein Bodenbereich des Kunststoffvorformlings beaufschlagt. Damit wird bevorzugt eine Außenoberfläche des Behältnisses mit dem Sterilisationsmedium beaufschlagt.

Bei einem bevorzugten Verfahren wird in einem Umgebungsbereich der Kunststoffvorformlinge aktiv eine Strömung des Sterilisationsmediums erzeugt. Unter "aktiv" wird dabei verstanden, dass diese Strömung insbesondere nicht oder nicht nur durch eine Bewegung der Kunststoffvorformlinge erzeugt wird, sondern bevorzugt zusätzlich weitere Maßnahmen vorgenommen werden, wie etwa eine Bewegung der Beaufschlagungseinrichtung, insbesondere relativ zu der Bewegung der Kunststoffvorformlinge und/oder eine Absolutbewegung der Beaufschlagungseinrichtungen oder der Einsatz eines Gebläses oder dergleichen.

Bei einem weiteren vorteilhaften Verfahren werden die Kunststoffvorformlinge während der Sterilisation durch einen Transportkanal transportiert, der den Transportpfad der Kunststoffvorformlinge wenigstens abschnittsweise umgibt. Vorteilhaft wird dabei dieser Transportkanal durch wenigstens zwei Wandungen gebildet, zwischen denen der Transportpfad der Kunststoffvorformlinge verläuft. Weiterhin kann eine dritte Wandung unterhalb des Transportpfades der Kunststoffvorformlinge vorgesehen sein.

Bei einem weiteren vorteilhaften Verfahren wird auch eine Innensterilisation der Kunststoffvorformlinge vorgenommen, bevorzugt also zumindest abschnittsweise auch eine Innenoberfläche der Kunststoffvorformlinge sterilisiert. Wie oben erwähnt kann dabei eine Innenoberfläche beispielsweise mit einem Sterilisationsmedium beaufschlagt werden, es wäre jedoch alternativ oder zusätzlich auch denkbar, dass diese Innenoberfläche mit einer Strahlung zum Zwecke der Sterilisation beaufschlagt wird.

Bevorzugt findet dabei diese Innensterilisation zumindest teilweise zeitgleich mit der Außensterilisation statt. Vorteilhaft laufen die Innensterilisation und die Außensterilisation wenigstens teilweise parallel zueinander ab. So wäre es möglich, dass die Kunststoffvorformlinge zeitgleich an ihren Außenoberflächen und ihren Innenoberflächen sterilisiert werden.

Weitere Vorteile und Ausführungsformen ergeben sich aus den beigefügten Zeichnungen.

Darin zeigen:
- Fig. 1: Eine schematische Darstellung zur Veranschaulichung der Erfindung;
- Fig. 2: eine erfindungsgemäße Vorrichtung einer ersten Ausführungsform;
- Fig. 3: eine erfindungsgemäße Vorrichtung in einer zweiten Ausführungsform;
- Fig. 4a-4e: fünf Darstellungen zur Veranschaulichung einer Beaufschlagung von Kunststoffvorformlingen mit einem Sterilisationsmedium; und
- Fig. 5: eine weitere Darstellung der Erfindung in einem Übergabebereich.

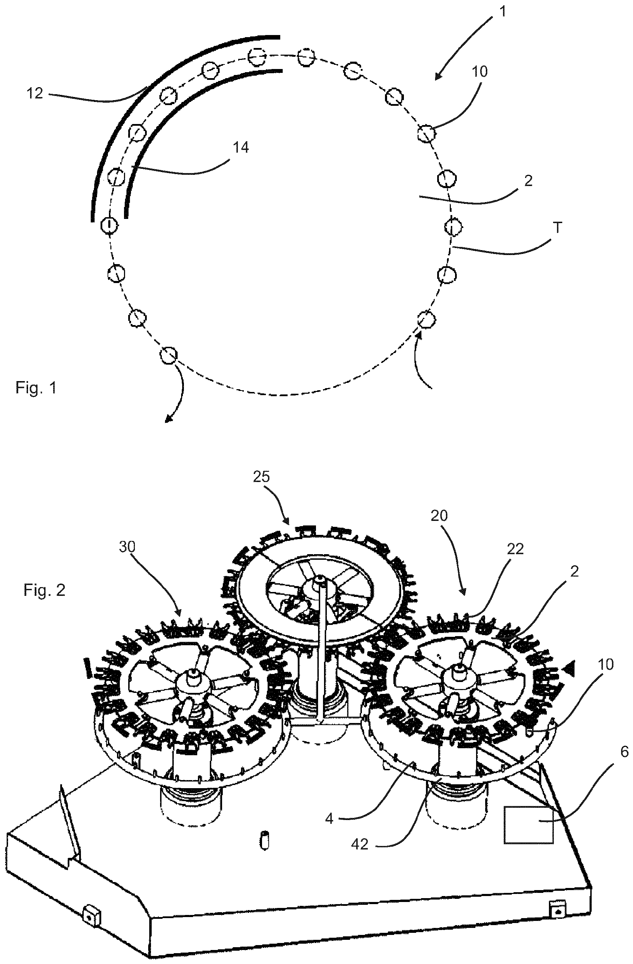

Fig. 1 zeigt eine schematische Darstellung zur Veranschaulichung der Erfindung. Dabei ist schematisch ein drehbarer Träger 2 dargestellt, mittels dem die Kunststoffvorformlinge 10 entlang ihres Transportpfades T bewegt werden. Die beiden unten stehenden Pfeile veranschaulichen eine Zuführung der Kunststoffvorformlinge und eine Abführung der Kunststoffvorformlinge von der erfindungsgemäßen Vorrichtung 1. Der drehbare Träger 2 stellt hier die Vorrichtung zum Sterilisieren der Kunststoffvorformlinge 10 dar und ist bevorzugt stromabwärts einer Erwärmungseinrichtung (nicht gezeigt) und stromaufwärts einer Nachfolgemaschine, wie z.B. einer Streckblasmaschine, (nicht gezeigt) angeordnet.

Der Transportpfad T verläuft zwischen zwei gekrümmten Wandungen 12 und 14, deren Krümmungen wiederum an die Krümmung des Transportpfades T angepasst sind. Dabei können diese Wandungen 12, 14 auch größere Bereiche des Transportpfades T abdecken, als hier in Fig. 1 gezeigt.

Fig. 2 zeigt eine schematische Darstellung einer erfindungsgemäßen Vorrichtung 1. Diese Vorrichtung weist hier zwei Sterilisationseinheiten 20 und 30 auf, wobei lediglich die rechts stehende mit Bezugszeichen versehen ist. Zwischen diesen beiden Sterilisationseinheiten 20, 30 ist eine Transporteinrichtung 25 vorgesehen, welche die Kunststoffvorformlinge von der ersten Sterilisationseinheit 20 zu der zweiten Sterilisationseinheit 30 fördert.

Erkennbar ist wieder der drehbare Träger 2, an dem eine Vielzahl von Halteeinrichtungen 22 zum Halten der Kunststoffvorformlinge 10 angeordnet ist. Bei diesen Halteeinrichtungen 22 handelt es sich hier um Greifklammern, welche die Kunststoffvorformlinge 10 in einem Bereich ihrer Mündung greifen. Bei diesen Halteeinrichtungen kann es sich insbesondere um steuerbare Halteeinrichtungen handeln, d.h. um Halteeinrichtungen, deren Öffnungszustand bzw. Schließzustand gesteuert werden kann.

Es ist jedoch nur ein Kunststoffvorformling 10 dargestellt. Das Bezugszeichen 4 kennzeichnet eine Beaufschlagungseinrichtung, die hier als Düse ausgebildet ist. Das Bezugszeichen 42 kennzeichnet einen Ringkanal, der die Vielzahl von Beaufschlagungseinrichtungen, die entlang des Transportpfades der Kunststoffvorformlinge 10 hintereinander angeordnet sind, mit dem Sterilisationsmedium versorgt. Bei der in Fig. 2 gezeigten Ausführungsform ist dieser Ringkanal 42 stationär angeordnet und die Kunststoffvorformlinge 10 bewegen sich diesem gegenüber.

Das Bezugszeichen 6 kennzeichnet grob schematisch eine Strömungserzeugungseinrichtung, die eine Strömung des Sterilisationsmediums erzeugt. Bei dieser Strömungserzeugungseinrichtung kann es sich beispielsweise um ein Gebläse oder dergleichen handeln.

Weiterhin weist die Vorrichtung bevorzugt eine (nicht gezeigte) Einhausung auf, sodass die Kunststoffvorformlinge innerhalb eines Gehäuses transportiert werden. Dabei wäre es möglich, dass der gesamte Innenraum als Reinraum ausgeführt ist, der gegenüber der Umgebung abgedichtet ist. Daneben können auch Beaufschlagungseinrichtungen vorhanden sein, welche diesen Reinraum mit einem Überdruck gegenüber einem (insbesondere atmosphärischen) Außendruck beaufschlagen.

Es wäre jedoch auch möglich, dass lediglich der Transportpfad der Kunststoffvorformlinge kanalartig eingehaust ist (nicht gezeigt). In diesem Falle könnte die Strömungserzeugungseinrichtung 6 beispielsweise an oder bei oder in einer der Wandungen, welche den Transportpfad der Kunststoffvorformlinge begrenzen, angeordnet sein.

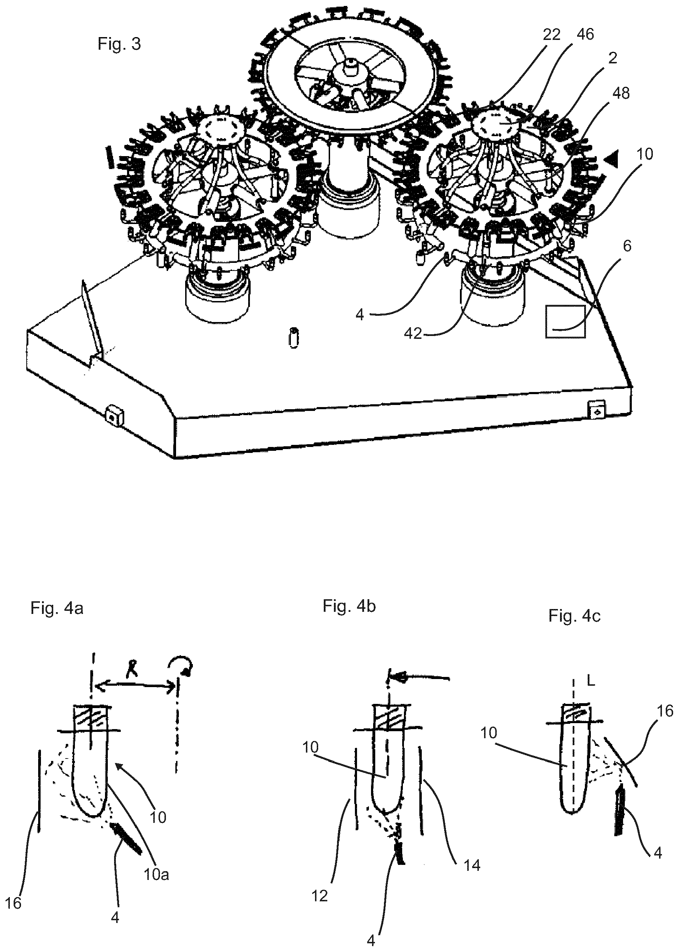

Fig. 3 zeigt eine weitere Ausführungsform der erfindungsgemäßen Vorrichtung. Bei dieser Ausführungsform sind die einzelnen Beaufschlagungseinrichtungen 4 sowie insbesondere auch deren Ringkanal 42 drehbar angeordnet. Dabei ist es, wie oben erwähnt, möglich, dass sich die Beaufschlagungseinrichtungen mit den Kunststoffvorformlingen mitbewegen, es kann jedoch auch eine andere Drehung, beispielsweise im umgekehrten Drehsinn, erzeugt werden. Das Bezugszeichen 46 kennzeichnet eine Verteileinrichtung, welche das Sterilisationsmedium auf den Kanal bzw. die einzelnen Beaufschlagungseinrichtungen verteilt. Dazu kann eine Vielzahl von Leitungen 48 vorgesehen sein, welche das Sterilisationsmedium zu den Beaufschlagungseinrichtungen 4 leiten. Daneben kann auch hier wieder die Strömungserzeugungseinrichtung 6 vorgesehen sein. Parallel oder auch (zumindest teilweise) zeitlich bzw. örtlich versetzt zur beschriebenen Außenentkeimung der Außenoberfläche der Kunststoffvorformlinge 10 kann auch eine Innenentkeimung stattfinden. Dies kann durch einen oberhalb der Mündung des Kunststoffvorformlings angeordneten Düsenring erfolgen, welcher das Sterilisationsmedium an die Innenwandungen der Kunststoffvorformlinge leitet. Das Einspeisen von Sterilisationsmedium für die Innen- sowie Außenentkeimung kann aus einem zentralen gemeinsamen Vorrat erfolgen.

Die Fig. 4a bis 4e zeigen mehrere Darstellungen, welche die Kunststoffvorformlinge 10 bzw. deren Außenoberflächen mit dem Sterilisationsmedium beaufschlagen. Bei der in Fig. 4a gezeigten Ausführungsform beaufschlagt die Beaufschlagungseinrichtung sowohl die Kunststoffvorformlinge unmittelbar, als auch eine Strömungsumlenkeinrichtung 16, wobei hier das Sterilisationsmedium abprallt, bzw. reflektiert wird und somit indirekt auf die Außenoberfläche 10a der Kunststoffvorformlinge gelangt.

Bei der in Fig. 4b gezeigten Ausführungsform sind wieder die beiden Wandungen 12 und 14 dargestellt, zwischen denen der Kunststoffvorformling transportiert wird. Dabei wäre es zusätzlich möglich, dass die Beaufschlagungseinrichtung 4 auch diese Wandungen 12 und 14 beaufschlagt und das Sterilisationsmedium von diesen Wandungen auch mittelbar auf die Kunststoffvorformlinge bzw. deren Außenwandung gelangt.

Fig. 4c zeigt eine weitere Darstellung der Beaufschlagung. Hier ist eine gegenüber der Längsrichtung L der Kunststoffvorformlinge 10 schräg angeordnete Umlenkeinrichtung 16 vorgesehen, die von der Beaufschlagungseinrichtung 4 beaufschlagt wird und auf diese Weise wiederum mittelbar die Kunststoffvorformlinge 10 beaufschlagt.

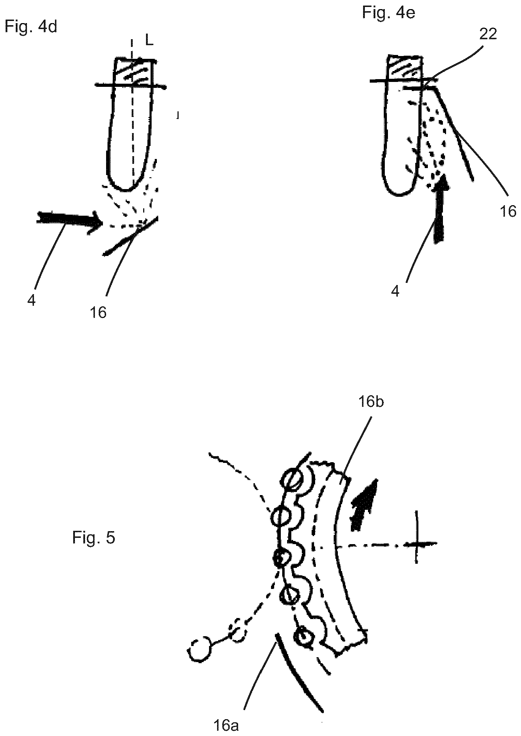

Fig. 4d zeigt eine weitere Ausführungsform der Beaufschlagung. Hier sind sowohl die Beaufschlagungseinrichtung 4 als auch die Umlenkungseinrichtung 16 unterhalb des Kunststoffvorformlings angeordnet und das Sterilisationsmedium wird von der Umlenkeinrichtung reflektiert und auf den Kunststoffvorformling geworfen. Zusätzlich wäre es möglich, dass Dreheinrichtungen vorgesehen sind, welche die Kunststoffvorformlinge 10 bezüglich ihrer eigenen Längsachse L drehen, sodass die Außenwandung der Kunststoffvorformlinge möglichst umfänglich mit dem Sterilisationsmedium beaufschlagt wird. Grundsätzlich wäre es auch denkbar, wenigstens eine Umlenkungseinrichtung 16 oder eine Beaufschlagungseinrichtung 4 in einem Bereich zwischen einer Drehachse des Trägers 2 und einer Längsachse des Kunststoffvorformlings 10, welcher von den Halteeinrichtungen 22 gehaltert wird, anzuordnen.

Bei der in Fig. 4e gezeigten Ausführungsform ist die Umlenkeinrichtung 16 mit den Beaufschlagungseinrichtungen 4 beweglich angeordnet, das heißt sie bewegt sich mit dieser mit. Weiterhin kann hier an der Umlenkeinrichtung 16 auch ein Haltemittel zum Halten der Kunststoffvorformlinge angeordnet sein.

Fig. 5 zeigt eine Darstellung zur Veranschaulichung der Übergabe der Kunststoffvorformlinge. Hier ist sowohl eine erste stationäre Umlenkeinrichtung 16a vorgesehen, als auch eine bewegliche Umlenkeinrichtung 16b, die hier gleichzeitig auch Haltemittel für die Kunststoffvorformlinge ausbildet. Dies bedeutet, dass hier sowohl die Umlenkeinrichtung 16a als auch die Umlenkeinrichtung 16b zur verbesserten Beaufschlagung der Kunststoffvorformlinge 10 mit dem Sterilisationsmedium beitragen.

Die Anmelderin behält sich vor, sämtliche in den Anmeldungsunterlagen offenbarten Merkmale als erfindungswesentlich zu beanspruchen, sofern sie einzeln oder in Kombination gegenüber dem Stand der Technik neu sind.

### Bezugszeichenliste

- 1: erfindungsgemäße Vorrichtung
- 2: drehbarer Träger
- 4: Beaufschlagungseinrichtungen
- 6: Strömungserzeugungseinrichtung
- 10: Kunststoffvorformlinge
- 10a: Außenoberfläche
- 12, 14: gekrümmte Wandungen
- 16: Strömungsumleitungseinrichtung, Umlenkeinrichtung
- 16a: erste Umlenkeinrichtung
- 16b: bewegliche Umlenkeinrichtung
- 20, 30: Sterilisationseinheiten
- 25: Transporteinrichtung
- 22: Halteeinrichtungen
- 42: Ringkanal
- 46: Verteileinrichtung
- 48: Vielzahl von Leitungen

- T: Transportpfad
- L: Längsachse

## Patentansprüche

1. Vorrichtung (1) zum Sterilisieren von Kunststoffbehältnissen (10) und insbesondere von Kunststoffvorformlingen (10), mit einer Transporteinrichtung (2), welche die Kunststoffbehältnisse (10) entlang eines vorgegebenen Transportpfades transportiert, mit wenigstens einer Beaufschlagungseinrichtung (4), welche eine Außenoberfläche der zu sterilisierenden Kunststoffbehältnisse mit einem fließfähigen Medium beaufschlagt,
**dadurch gekennzeichnet, dass**
die Beaufschlagungseinrichtung derart angeordnet ist, dass sie einen vorgegebenen, unterhalb einer Mündung der Kunststoffvorformlinge liegenden Bereich der Kunststoffbehältnisse (10) mit dem Sterilisationsmedium beaufschlagt, wobei die Vorrichtung (1) eine Strömungserzeugungseinrichtung (6) aufweist, welche in dem Bereich des Transportpfades und an der Aussenoberfläche der Kunststoffbehältnisse (10), insbesondere an der Aussenoberfläche der Kunststoffvorformlinge, eine Strömung des fließfähigen Mediums erzeugt.

2. Vorrichtung (1) nach Anspruch 1,
**dadurch gekennzeichnet, dass**
die Strömungserzeugungseinrichtung (6) dazu eingerichtet und dafür vorgesehen ist, dass in der Umgebung der Kunststoffbehältnisse (10) eine, insbesondere turbulente, Strömung des Sterilisationsmediums erzeugbar ist, sodass ein Entkeimungsvorgang dadurch verbessert wird, dass ein Stofftransport durch eine gezielte Umströmung der zu entkeimenden Außenoberflächen der Kunststoffbehältnisse verbessert wird.

3. Vorrichtung (1) nach dem vorhergehenden Anspruch,
**dadurch gekennzeichnet, dass**
diese turbulente Strömung durch einen Ventilator erzeugt wird, der sich in einem Behandlungsbereich befindet und in Richtung des Transportpfades der Kunststoffbehältnisse (10) ausgerichtet ist, und/oder ein entsprechendes Gebläse zum Einsatz kommt, welches diese Strömung erzeugt.

4. Vorrichtung (1) nach dem vorhergehenden Anspruch,
**dadurch gekennzeichnet, dass**
der Ventilator und/oder das Gebläse einen separaten Antrieb aufweist, der sich bevorzugt außerhalb des Behandlungsbereiches befindet.

5. Vorrichtung (1) nach Anspruch 3 oder 4,
**dadurch gekennzeichnet, dass**
eine Antriebsleistung des Ventilators und/oder des Gebläses über ein entsprechendes Getriebe von einer Drehbewegung eines Transportsterns und/oder der Transporteinrichtung abgezweigt wird.

6. Vorrichtung (1) nach zumindest einem der vorhergehenden Ansprüche,
**dadurch gekennzeichnet, dass**
die Beaufschlagungseinrichtung (4) unterhalb des Transportpfades (T) der Kunststoffvorformlinge angeordnet ist.

7. Vorrichtung (1) nach zumindest einem der vorhergehenden Ansprüche,
**dadurch gekennzeichnet, dass**
die sich die Beaufschlagungseinrichtung (4) in Transportrichtung der Kunststoffvorformlinge (10) mit diesen mitbewegt.

8. Vorrichtung (1) nach wenigstens einem der vorangegangenen Ansprüche,
**dadurch gekennzeichnet, dass**
die Vorrichtung eine erste Wandung (12) aufweist, welche sich in einer Richtung des Transportpfades erstreckt und welche bevorzugt seitlich des Transportpfades (T) angeordnet ist.

9. Vorrichtung (1) nach zumindest einem der vorhergehenden Ansprüche,
**dadurch gekennzeichnet, dass**
die Vorrichtung eine zweite Wandung (14) aufweist, wobei der Transportpfad (T) der Kunststoffvorformlinge zwischen der ersten Wandung (12) und der zweiten Wandung (14) angeordnet ist.

10. Vorrichtung (1) nach wenigstens einem der vorangegangenen Ansprüche,
**dadurch gekennzeichnet, dass**
die Vorrichtung eine Umlenkeinrichtung aufweist, welche eine Strömung des fließfähigen Mediums umlenkt.

11. Verfahren zum Sterilisieren von Kunststoffvorformlingen (10), wobei die Kunststoffvorformlinge (10) entlang eines vorgegebenen Transportpfads (T) transportiert werden und während dieses Transports wenigstens ein Bereich einer Außenoberfläche (10a) des Kunststoffvorformlings (10) mit einem fließfähigen Sterilisationsmedium beaufschlagt wird,
**dadurch gekennzeichnet, dass**
von wenigstens einer Beaufschlagungseinrichtung (4) wenigstens ein Bereich der Außenoberfläche der Kunststoffvorformlinge beaufschlagt wird, der unterhalb eines Mündungsbereichs der Kunststoffvorformlinge (10) liegt, wobei die Vorrichtung (1) eine Strömungserzeugungseinrichtung (6) aufweist, welche in dem Bereich des Transportpfades und an der Aussenoberfläche der Kunststoffbehältnisse (10), insbesondere an der Aussenoberfläche der Kunststoffvorformlinge, eine Strömung des fließfähigen Mediums erzeugt.

12. Verfahren nach Anspruch 11,
**dadurch gekennzeichnet, dass**
in einem Umgebungsbereich der Kunststoffvorformlinge (10) aktiv eine Strömung des Sterilisationsmediums erzeugt wird.

13. Verfahren nach Anspruch 11,
**dadurch gekennzeichnet, dass**
die Strömung des Sterilisationsmediums turbulent ist.

14. Verfahren nach wenigstens einem der vorangegangenen Ansprüche 11 - 13,
**dadurch gekennzeichnet, dass**
die Kunststoffvorformlinge während ihrer Sterilisation durch einen Transportkanal transportiert werden, der den Transportpfad (T) der Kunststoffvorformlinge wenigstens abschnittsweise umgibt.

15. Verfahren nach wenigstens einem der vorangegangenen Ansprüche 11 - 13,
**dadurch gekennzeichnet, dass**
auch eine Innenoberfläche der Kunststoffvorformlinge sterilisiert wird, wobei bevorzugt die Außensterilisation der Kunststoffvorformlinge und die Innensterilisation der Kunststoffvorformlinge wenigstens teilweise zeitgleich vorgenommen wird.
